Europäisches Patentamt

European Patent Office

Office européen des brevets

(11) Publication number: 0 319 638
A1

# (12) EUROPEAN PATENT APPLICATION

(21) Application number: 88105054.6

(22) Date of filing: 29.03.88

(51) Int. Cl.⁴: **A61K 7/48 , A61K 9/50**

(30) Priority: 08.12.87 US 129829

(43) Date of publication of application:
14.06.89 Bulletin 89/24

(84) Designated Contracting States:
BE CH DE FR GB IT LI LU NL

(71) Applicant: ESTEE LAUDER INC.
767 Fifth Avenue
New York New York 10022(US)

(72) Inventor: Vichroski, Thomas J.
29 Cedar Place
Oakdale New York 11769(US)
Inventor: Cioca, Gheorghe
3 West Cliff Lane
Lake Grove New York 11755(US)
Inventor: Smith, Walter P.
89 Blackberry Drive
Stamford Connecticut 06903(US)
Inventor: Hayward, James A.
4 Chuck Court
Port Jefferson New York 11777(US)

(74) Representative: Vossius & Partner
Siebertstrasse 4 P.O. Box 86 07 67
D-8000 München 86(DE)

(54) Liposome containing cosmetic and pharmaceutical compositions and methods for utilizing such compositions.

(57) A composition and method for restoring the skin's natural barrier to moisture loss, thereby enhancing the ability of the skin to retain its moisture. The composition comprises a liposome, wherein the liposome comprises a cerebroside, a phospholipid and a cholesteric material. There is further disclosed cosmetic compositions and methods for treating the skin comprising a multiple barrier delivery system for applying a biologically active material to the skin, the delivery system comprising a liposome, an encapsulated biologically active material, and a starch-grafted polyacrylic acid gel.

EP 0 319 638 A1

EP 0 319 638 A1

# LIPOSOME CONTAINING COSMETIC AND PHARMACEUTICAL COMPOSITIONS AND METHODS FOR UTILIZING SUCH COMPOSITIONS

This invention relates to liposome containing compositions and methods for using such compositions. The liposome compositions of the invention are useful for making cosmetic and pharmaceutical compositions for application to the skin, particularly treatments for moisturizing and restoring the youthful appearance of the skin.

In cosmetic and pharmaceutical formulations for the skin, it is known to use various delivery systems to control the release of biologically active materials to the skin. Liposomes have been used for this purpose in a variety of products.

Liposomes are microscopic vesicles, generally spherically shaped, formed from one or several concentric layers (lamellae) of lipid molecules (i.e., compounds having a lipophobic hydrophilic moiety and a lipophilic hydrophobic moiety). See US-A-4,224,179. For a general discussion of liposomes, reference is made to Gregoriadis et al. (Eds.), Liposomes in Biological Systems, John Wiley & Sons, N.Y. (1980).

In use, a liposome fuses or attaches to a cell wall, where its payload, i.e., the biologically active material encapsulated within the liposome, may be released and become part of the cell. To function effectively as a delivery vehicle for biologically active materials, the liposome should be sufficiently stable so that its payload does not escape prior to the fusion of the liposome to the cell wall. On the other hand, the liposome should readily release its payload upon interaction with the cell.

Cosmetic and pharmaceutical compositions for applying biologically active materials to the skin typically include a carrier for the active materials in the composition. For example, the carrier may be selected so that the composition takes the form of an emulsion, a cream or a gel. A suitable carrier desirably possesses certain characteristics depending on the nature of the active material. For example, the carrier may protect the active material from degradation by electrolytes or luminous radiation and, thus, stabilize the composition. Such carriers will preferably possess characteristics that provide a comfortable and pleasing effect to the skin during application.

The selection of a suitable carrier for use in compositions in which the release of biologically active materials is controlled is complicated by the fact that the carrier must be chosen such that it does not interfere with (and preferably enhances) the ability of the composition to release its active material at a suitable rate.

One object of the present invention is to provide compositions useful for cosmetic or pharmaceutical use that comprise an improved liposome.

Another object of this invention is to provide an improved composition for the controlled delivery of biologically active materials to the skin.

Yet another object of this invention is to provide a composition and method for restoring the skin's natural barrier to moisture loss, thereby enhancing the ability of the skin to retain its natural moisture.

A further object of this invention is to provide an improved carrier for liposome compositions in which the liposome encapsulates a biologically active material.

This invention achieves these objects by providing, in one embodiment, a composition comprising a biologically active material (e.g., trehalose) encapsulated within a liposome, the liposome being suspended in a gel matrix comprising a starch-grafted polyacrylic acid.

The liposomes of this invention preferably comprise a cerebroside, a phospholipid and a cholesteric material. We believe that hydrogen bonding between the components of the liposome stabilizes the liposome in the lamellar phase so as to prevent leakage of the encapsulated biologically active material. The liposomes of the present invention are compatable with the cells of the skin, and release their payload of active materials into skin cells in a desirable manner. In addition, the liposomes of the invention, by themselves, serve to restore the skin's natural barrier to prevent moisture loss from the skin.

As used herein, the term "biologically active material" means any substance suitable for topical administration to the skin that induces a desired cosmetic or pharmaceutical effect in the skin. For example, the biologically active material may be selected from the group consisting of antibiotics, anti-inflammatory agents, rubefacients, sunscreens, emolients, vitamins and skin protectants. The composition of the invention may take the form of cosmetic formulations such as hand creams, acne products, deodorants, antiperspirants, body powders, lip ices, lip sticks, baby lotions, medicated facial creams, shampoos, shaving creams, pre- and after-shave lotions and hairgrooming preparations.

As used herein, the term "carrier" shall include materials that are suitable for use in contact with the skin and which, when combined with the liposome and biologically active materials of the invention, take the

2

form of a liquid, gel, fluid ointment, cream, lotion or the like.

The preferred carrier for use in this invention comprises a starch-grafted polyacrylic acid gel. We believe that this preferred carrier is particularly effective when used in conjunction with liposomes containing biologically active materials because it effectively limits liposome-liposome interactions. Such interactions can lead to fusion of the liposomes to themselves, with resulting premature leakage of the encapsulated biologically active materials. The preferred carrier of the present invention is also comfortable and pleasant to use.

We believe that the starch-grafted polyacrylic acid gel forms a polymeric skin around the liposomes, which skin serves to protect the individual liposomes from liposome-liposome interaction. We further believe that this skin also functions to provide a second barrier (the first barrier being the liposome itself) to impede the release of the biologically active materials to the skin, thereby enhancing the controlled release of biologically active materials to the skin.

The compositions of the invention may be applied in effective amounts to the skin in any suitable manner. The amount applied and the frequency of application will, of course, vary depending on the composition being applied to the skin and the effect desired.

The liposomes of the present invention preferably comprise at least 30% by weight of cerebroside based on the total weight of the cerebroside, phospholipid and cholesteric materials in the liposome. A suitable cerebroside for use in the invention can be obtained from Canada Packers, Toronto, Canada.

A variety of phospholipids may be used to make the liposome of the invention. Phosphatidyl choline (lecithin) and sphingomyelin, which can also be obtained from Canada Packers, are preferred phospholipids for use in the present invention.

Cholesterol is the preferred cholesteric material for use in making the liposomes of the invention. Cholesterol derivatives such as plant sterols and steroids such as hydroxy cortisone may also be used.

An antioxidant may also be incorporated into the liposome. Suitable antioxidants include vitamin E and its derivatives, BHT and BHA.

Numerous techniques for making liposomes, including cerebroside containing liposomes, are known in the art. In general, these techniques involve mixing the components that form the liposome, as well as any components that are to be encapsulated within the lipsome, under conditions that permit the formation of the liposome.

We prefer to process the raw materials used to make the liposome of the present invention in a microfluidizer. This permits the mass manufacture of liposomes that are stable in the lamellar phase. In other words, microfluidization permits the formation of liposomes comprising a cerebroside, a phospholipid and a cholesteric material that retain an ordered lipid bilayer during storage. Microfluidizers suitable for use in making the compositions of our invention are available from Microfluidics Corp. as MFC microfluidizers and the use of such microfluidizers to make liposomes is described in Mayhew et al., "Characterization of Liposomes Prepared Using a Microemulsifier," Biochem. et Biophys. Acta., 775, 169-74 (1984).

The specific method for producing the liposome and encapsulating an active substance therein is not critical. Various methods in addition to microfluidization are known in the art, including, e.g., ultrasonification, formation of reverse phase evaporation vessels, hydration of thin films, and detergent dialysis.

The liposomes of this invention may be used to encapsulate and serve as delivery vehicles for any biologically active material. The preferred material is a saccharide, most preferrably trehalose. The trehalose in the composition of the invention functions to increase the fluidity of membranes, thereby serving as a moisturizing agent for the skin. In addition, the trehalose stablizes the liposomes when they are freeze-dried and rehydrated. Such freeze-drying and rehydration may be utilized when it is desired to store the liposomes of the invention for an extended period of time.

In addition, we believe that the trehalose stablizes the liposome membrane structure by interacting with phospholipid head groups in the liposome. Such stabilization may be due to an increase in the fluidity of the acyl moieties of the phospholipid head group.

The preferred carrier for the liposomes of the invention are gel matrices that form a polymeric lattice around the liposomes. Liposomes tend to destabilize when they collide with and fuse to each other. We believe that such polymeric lattices, in effect, form a skin around the liposome, thereby stabilizing the liposome by limiting diffusion and liposome-liposome interaction.

We believe that starch grafting results in the formation of numerous cross links, which in turn allows the gelling agent to retain a large volume of water. This water tends to further stabilize the liposomes. While we believe that a starch-grafted polyacrylic acid carrier will have this stabilizing affect on any liposome (including liposomes that encapsulate a biologically active material), in the most preferred embodiment of this invention we use this carrier in combination with the liposomes comprising a cerebroside, a phospholipid and a cholesteric material.

Although not part of the invention, the compositions of th
biological extracts, e.g., collagens, and vitamins. A composition (
is described in the example below.

The carrier of this invention may also have incorporated
suitable for cosmetic or pharmaceutical use. For example, polyac..
the carrier as additional gelling agents. A fragrance may be included. .
also be added as a viscosity builder. Similarly, substances such as dye.
employed to enhance the appearance of the compositions of the invention.

The following example is presented for the sole purpose of further illustrating the p.
is not to be taken as limiting thereto. Unless otherwise specified, all parts and percentages are

## Example

This example illustrates the production of a liposome, trehalose and starch-grafted polyacrylic acid
containing product having the following composition:

| Component | % by weight |
| --- | --- |
| 0.2% Starch-Grafted Polyacrylic Acid Solution | 21.0000 |
| Liposome Phase | 10.0000 |
| Carrier Gel Additives | 69.0000 |

## 1. Preparation of Starch-Grafted Polyacrylic Acid Solution

We first prepare an aqueous solution of starch-grafted polyacrylic acid by pouring deionized water into
a mixing kettle. While mixing the deionized water at 150-200 rpm using a Lightnin mixer, we add a
preservative such as methyl paraban. We continue mixing until uniform. We then slowly add Sanwet (Sanyo
Chemical Co.) starch-grafted polyacrylic acid while mixing at 300-350 rpm. We continue mixing for
approximately 2-3 hrs. The resulting mixture, which contains 0.5% preservative and 0.2% Sanwet, can be
stored for up to 5 days.

## 2. Preparation of Liposome Phase

We next prepare a liposome phase having the following approximate composition:

| Component | % by weight |
| --- | --- |
| Deionized water | 87.4125 |
| Trehalose | 10.0000 |
| Triethanolamine 99% | 0.0500 |
| Lipid mixture | 2.5000 |
| Phosphatidyl choline 32.900 | |
| Sphingomyelin 5.0000 | |
| Cholesterol 1.1000 | |
| Cerebroside 61.0000 | |
| Fragrance | 0.0375 |

The ingredients are added and mixed together in the order listed, using vigorous stirring. We then
microfluidize the mixture using an MFC microluidizer.

### 3. Combination of the Starch-Grafted polyacrylic Acid Solution, the Liposome Phase and the Gel Additives

We first add to our starch-grafted polyacrylic acid solution dimethicones or equivalent lubricants (13.0000%), a viscosity builder such as glyceryl monostearate, propylene glycol monostearate or polyethelene glycol and the like (4.0000%) and a chelating agent such as Sequestrene NA3T (0.0700%) in a steam-jacketed primary kettle. These materials are added in the amount necessary to obtain a final product having the stated weight percentages of the materials. Unless otherwise stated, the percentage of each material stated hereunder is stated in terms of the percentage of the material in the final product.

We heat the resulting mixture to 60°C, while mixing at 250-300 rpm using a Lightnin mixer. We continue mixing until the mixture is uniform, at which time we stop heating and add a polyacrylic gelling agent such as Carbopol (15.0000%) while mixing at 300-350 rpm until the gelling agent is completely dispersed.

We then cool the mixture to 40°C at a rate of 1°C per minute while agitating it slowly. At 40°C, we add triethanolamine 99 organic base (0.1500%) to the mixture under continued slow agitation and continued cooling at 1°C per minute. At 30°C, we stop cooling.

In an auxiliary mixing vessel, we combine a collagen mixture (5.0000%) and Germall (0.1740%), while mixing at 1500-2000 rpm until the solution is smooth and particle-free. A collagen mixture is supplied by Chemisches Laboratorium Dr. Kurt Richter GmbH, Berlin, West Germany. The collagen-Germall mixture is combined with the liposome phase (10.0000%), FD&C Blue No. 1 (0.10000% aqueous solution) (0.0300%), and the ingredients already in the primary kettle and mixed to uniformity.

Into an auxiliary mixing vessel, we combine chiral nematic liquid crystals comprised of a mixture of benzoic acid esters with Vitamin E acetate and cholesteric esters blended to yield a specific colored display. Using a hand paddle, we stir the resulting mixture until it is uniform.

We prepare an aqueous solution containing 0.7500% by weight TEA-acrylamid copolymer, based on the weight of the solution and then add that solution into a support kettle such that the solution comprises 26.7120% by weight of the final product. We mix the resulting solution with a Lightnin mixer for about 5 minutes.

We then combine the liquid crystal mixture (4.7640%) from the auxiliary mixing vessel with the TEA-polyacrylamid solution and mix the resulting mixture in the support kettle with a Lightnin mixer at 150-200 rpm, until the liquid crystal particles are uniformly dispersed (about 2-3 minutes). We then add Vitamin E acetate (0.2000%) to the resulting mixture and slowly mix until the mixture is uniform.

We next add the mixture from the support kettle to the starch-grafted polyacrylic acid and liposome-containing composition in the primary mixing kettle while slowly mixing. When the addition is complete, we continue slow mixing for approximately 5 minutes until the mixture is uniform.

## Claims

1. A composition comprising a liposome, wherein the liposome comprises a cerebroside, a phospholipid and a cholesteric material.

2. The composition of claim 1, further comprising a biologically active material that is encapsulated within the liposome.

3. The composition of claim 2, wherein the cerebroside comprises at least about 30% by weight of the total weight of the cerebroside, phospholipid and cholesteric material.

4. The composition of claim 2, further comprising a carrier.

5. The composition of claim 4, wherein the carrier comprises a starch-grafted polyacrylic acid.

6. The composition of claim 3, wherein the biologically active material is trehalose.

7. A composition comprising a biologically active material, a liposome and a starch-grafted polyacrylic acid gel, wherein the biologically active material is encapsulated within the liposome and the liposome is suspended in the gel.

8. The composition of claim 7, wherein the biologically active substance is trehalose.

9. The composition of claims 7 and 8, wherein the liposome comprises a cerebroside, a phospholipid and a cholesteric material.

10. A method for restoring a youthful appearance to the skin, said method comprising applying to the skin an effective amount of the composition of any one of claims 2 to 9.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | EP-A-0 036 277 (THE REGENTS OF THE UNIVERSITY OF CALIFORNIA) * Page 4, line 20 - page 8, line 3; page 13, table II, no. 4 * | 1-4 | A 61 K 7/48 A 61 K 9/50 |
| Y | | 6,9,10 | |
| X | CHEMICAL ABSTRACTS, vol. 89, no. 13, 25th September 1978, pages 497-498, abstract no. 104517c, Columbus, Ohio, US; M.M. JONAH et al.: "Tissue distribution of EDTA encapsulated within liposomes containing glycolipids or brain phospholipids", & BIOCHIM. BIOPHYS. ACTA 1978, 541(3), 321-33 * Abstract * | 1,2,4 | |
| Y | FR-A-2 315 991 (L'OREAL) * Page 6, line 1 - page 7, line 23; page 9, example 1; page 13, example 12 * | 4,5,7,8 ,10 | |
| Y | WO-A-8 601 103 (THE LIPOSOME CO., INC.) * Front page; abstract * | 6,8 | TECHNICAL FIELDS SEARCHED (Int. Cl.4) A 61 K |
| Y | EP-A-0 105 657 (BEECHAM INC.) * Pages 6,7, examples 6,7; claims 1-4 * | 4,5,7- 10 | |
| A | EP-A-0 199 362 (MASSACHUSETTS INSTITUTE OF TECHNOLOGY) | | |

The present search report has been drawn up for all claims

| Place of search | Date of completion of the search | Examiner |
|---|---|---|
| THE HAGUE | 10-03-1989 | BENZ K.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P0401)